# EUROPEAN PATENT APPLICATION

(11) **EP 4 778 567 A1**
(43) Date of publication of application: **22.07.2026**
(21) Application number: 26151872.4
(22) Date of filing: 14.01.2026
(51) Int. Cl.: A61N 1/372, A61B 17/34, A61B 17/00, A61N 1/375, A61M 25/00

(54) **BIOSTIMULATOR TRANSPORT SYSTEM HAVING VENTRICLE-SHAPED EXPANDABLE STRUCTURE**

(30) Priority: 15.01.2025 US 202563745752 P; 13.01.2026 US 202619447668
(71) Applicant: Pacesetter, Inc., Sylmar, CA 91342 (US)
(72) Inventor: Arnar, Bernhard, Sylmar, CA 91342 (US); Jackson, Aaron, Sylmar, CA 91342 (US)
(74) Representative: Barker Brettell Sweden AB

(57) **Abstract**

A biostimulator transport system (302) includes a sheath (312) having a distal sheath end (314). The biostimulator transport system (302) includes a deployment tube (316) extending from the distal sheath end (314) to a distal tube end (318). The biostimulator transport system (302) includes an expandable structure (700) having a ventricle-shaped envelope (904) surrounding the deployment tube (316). The ventricle-shaped envelope (904) includes a front boundary (702) coupled to the distal tube end (318) and a rear boundary (704) coupled to the distal sheath end (314). The front boundary (702) and the rear boundary (704) are joined along one or more of a bottom edge (710) and lateral edges (802, 804).

## Description

### TECHNICAL FIELD

The present disclosure relates to biostimulators and related biostimulator systems. More specifically, the present disclosure relates to leadless biostimulators and related systems useful for septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Pacing at the left bundle branch area (LBBAP) is an alternative to His-bundle pacing. LBBAP involves pacing past the His-bundle toward the right ventricular apex. More particularly, a pacing site for LBBAP pacing is typically below the His-bundle, on the interventricular septal wall. LBBAP can prevent pacing induced cardiomyopathy in a bradycardia patient population. LBBAP can also be an effective alternative to cardiac resynchronization therapy in treating heart failure patients. To achieve optimal results, the pacing site for physiological LBBAP can be high on the interventricular septal wall, in the region close to the tricuspid valve and pulmonary artery outflow track. Furthermore, the pacing site may be at a depth of up to 1.5 cm within the septal wall.

### SUMMARY

Significant challenges are associated with delivering a leadless cardiac pacemaker to the left bundle branch area pacing (LBBAP) site. For example, the leadless cardiac pacemaker may be required to be delivered through limited available space within the right ventricle to engage a particular location at a particular angle and extend deep, e.g., 1 cm, into the septal wall. Several attempts are often required to achieve such septal wall engagement because existing leadless pacemakers may not fit, or may interfere with heart structures, when placed at the optimal pacing site for LBBAP. Furthermore, a shape of the septal wall can misdirect or bias the leadless cardiac pacemaker into anatomical structures, e.g., posterior and anterior ventricular grooves, which are not at the target location. Thus, there is a need for a leadless biostimulator transport system that can deliver a leadless cardiac pacemaker to engage the interventricular septal wall at a target location and/or angle such that the biostimulator can penetrate deep into the septal wall to pace the LBB directly for improved conduction system capture and battery life.

A biostimulator transport system is described. In an embodiment, the biostimulator transport system includes a sheath having a distal sheath end. The biostimulator transport system includes a deployment tube extending from the distal sheath end to a distal tube end. The biostimulator transport system includes an expandable structure having a ventricle-shaped envelope surrounding the deployment tube. The ventricle-shaped envelope includes a front boundary coupled to the distal tube end and a rear boundary coupled to the distal sheath end. The front boundary and the rear boundary are joined along one or more of a bottom edge and lateral edges.

A biostimulator system is described. In an embodiment, the biostimulator system includes a biostimulator movable through the biostimulator transport system. A method of delivering the biostimulator to a target anatomy using the biostimulator transport system is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a side view of a biostimulator, in accordance with an embodiment.
FIG. 3 is a perspective view of a biostimulator system in an undeployed state, in accordance with an embodiment.
FIG. 4 is a diagrammatic cross section of a patient heart illustrating an example delivery of a biostimulator to a septal wall, in accordance with an embodiment.
FIG. 5 is a diagrammatic cross section of a patient heart illustrating a ventricle shape, in accordance with an embodiment.
FIG. 6 is a pictorial view of a virtual model of a ventricle shape, in accordance with an embodiment.
FIG. 7 is a diagrammatic cross section of a patient heart illustrating an example delivery of a biostimulator through an expandable structure of a biostimulator transport system, in accordance with an embodiment.
FIG. 8 is a diagrammatic cross section of a patient heart illustrating an example delivery of an expandable structure of a biostimulator transport system in a ventricle, in accordance with an embodiment.
FIG. 9 is a perspective view of a biostimulator transport system in an expanded state, in accordance with an embodiment.
FIG. 10 is a perspective cross-sectional view of a biostimulator transport system in an expanded state, in accordance with an embodiment.
FIG. 11 is a perspective view of a biostimulator transport system in an expanded state, in accordance with an embodiment.
FIG. 12 is a perspective view of a biostimulator transport system in an expanded state, in accordance with an embodiment.
FIG. 13 is a perspective view of a biostimulator transport system in an unexpanded state, in accordance with an embodiment.
FIG. 14 is a flowchart of a method of implanting a biostimulator in a target tissue, in accordance with an embodiment.

### DETAILED DESCRIPTION

Embodiments describe a biostimulator transport system for delivering a biostimulator to perform pacing, e.g., septal pacing in a ventricle. The biostimulator may, however, be used in other applications, such as atrial wall pacing. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator, a biostimulator transport system, or a biostimulator system. Similarly, "proximal" may indicate a second direction opposite to the first direction. Such terms are provided to establish relative frames of reference, however, and are not intended to limit the use or orientation of a biostimulator, a biostimulator transport system, or a biostimulator system to a specific configuration described in the various embodiments below.

In an aspect, a biostimulator transport system includes an expandable structure having a target anatomy shaped, e.g., ventricle-shaped, envelope that can be deployed to engage anatomical landmarks. The biostimulator transport system can utilize the anatomical landmarks to orient a deployment tube extending through the expandable structure to a target location. For example, when expanded in a ventricle, a distal tube end of the deployment tube can be positioned at the target location on a septal wall. A biostimulator can be advanced through the deployment tube into the septal wall at the target location to perform tissue pacing. More particularly, the biostimulator transport system can guide the biostimulator into a correct position and angle at the target location along the septal wall using anatomical landmarks, such as ventricular grooves, to help orient the expandable structure.

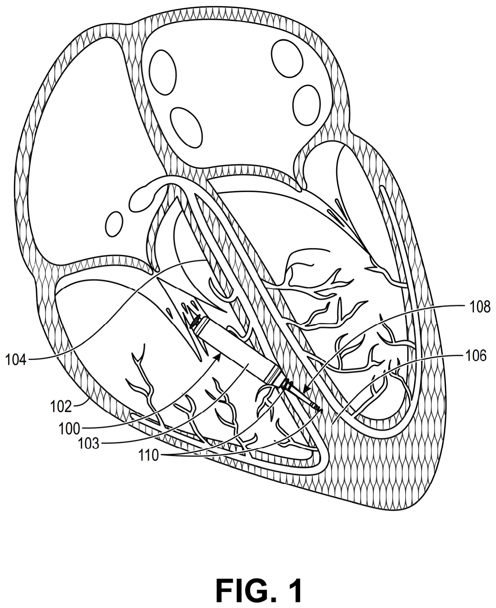

Referring to FIG. 1, a diagrammatic cross section of a patient heart illustrating an example implantation of a biostimulator in a target anatomy is shown in accordance with an embodiment. The diagram shows a biostimulator 100 attached to a patient heart 102 with a body 103 of the biostimulator positioned at an appropriate location and angle along a septal wall 104. More particularly, the location and the angle facilitates effective engagement or stimulation of a target tissue 106, e.g., a left bundle branch, by a pacing element 108.

The biostimulator 100 can be leadless (and thus, may be a leadless cardiac pacemaker). The biostimulator 100 can be attached to the septal wall 104 of the heart 102. More particularly, the biostimulator 100 can be delivered to the septum 104, and one or more elements, such as a fixation element 110 can pierce the septal wall to engage and anchor the biostimulator 100 to the tissue. For example, the fixation element 110 can include a fixation helix or fixation tines. In a particular embodiment, the biostimulator 100 can use two or more electrodes located on or within a housing of the biostimulator 100, e.g., the pacing element 108 and/or an outer housing surface of the housing, for pacing the cardiac chamber upon receiving a triggering signal from at least one other device within the body. More particularly, in an embodiment, one or more of the fixation element 110 or the pacing element 108 is an active electrode.

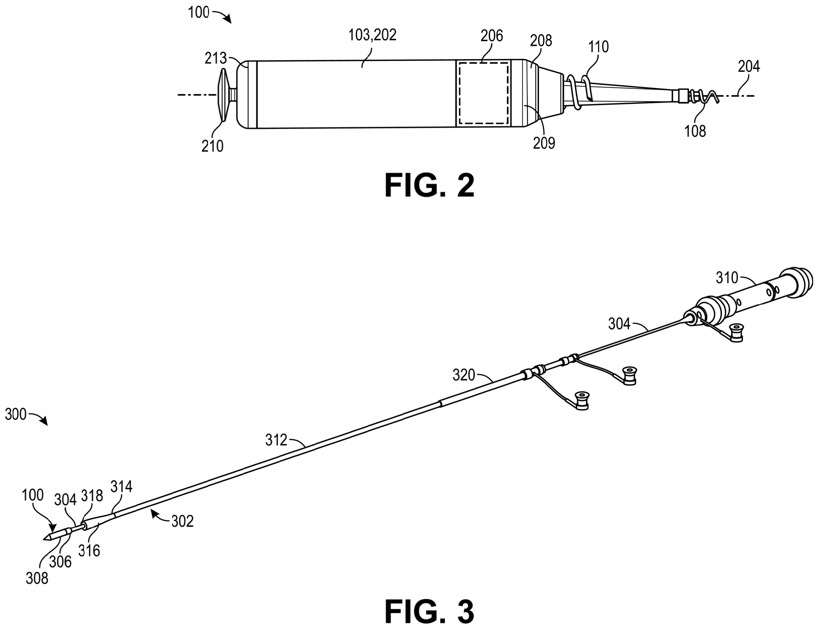

Leadless pacemakers or other leadless biostimulators can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems, and are described further below. For example, in some implementations of transport systems (FIG. 3), a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 110, the transport system can include a biostimulator coupling, e.g., a docking cap or key, at a distal end of the catheter, and the docking cap or key may be configured to engage the leadless pacemaker and apply torque to screw the active engaging mechanism into or out of the tissue. In other implementations, the biostimulator coupling of the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 110 may be positioned for deep septal pacing at respective bundle branches in the septum 104. For example, an active electrode of the pacing element 108 can be positioned at the left bundle branch in the septum 104. Similarly, the fixation element 110 can be positioned at the right bundle branch in the septum 104. Optionally, one of the elements may be at a bundle branch and the other element may not be at a bundle branch.

Referring to FIG. 2, a side view of a biostimulator is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the pacing element 108 that acts as an active electrode and/or a portion of the fixation element 110 or the body 103 that acts as an active electrode. The electrodes can deliver pacing pulses to bundle branches within the septum 104 of the heart 102 to perform deep septal pacing, and optionally, can sense electrical activity from the muscle. The electrodes may also communicate bidirectionally with at least one other device within or outside the body.

In an embodiment, the body 103 of the biostimulator 100 includes a housing 202 having a longitudinal axis 204. The housing 202 can contain a primary battery to provide power for pacing, sensing, and communication, which may include, for example, bidirectional communication. The housing 202 can optionally contain an electronics compartment 206 (shown by hidden lines) to hold circuitry adapted for different functionality. For example, the electronics compartment 206 can contain circuits for sensing cardiac activity from the electrodes, circuits for receiving information from at least one other device via the electrodes, circuits for generating pacing pulses for delivery to tissue via the electrodes, or other circuitry. The electronics compartment 206 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 110 coupled to the housing 202. The fixation element 110 can include a helical fixation element and/or several tines. More particularly, the biostimulator 100 can include a header assembly having a flange 208 coupled to a distal housing end 209 of the housing 202, and the fixation element 110 can be coupled to and extend distal to the flange 208. The fixation element 110 can extend about the longitudinal axis 204. For example, in the case of a helical fixation element, the element can spiral about the longitudinal axis 204 to a distal tip. In the case of a fixation element 110 having several tines, the tines can be arranged about the longitudinal axis 204 and extend to respective distal tips. Accordingly, when the biostimulator 100 is delivered to the target tissue, the distal tip(s) of the fixation element 110 can pierce the tissue and the housing 202 can be rotated or pushed to affix the fixation element 110 to the target tissue.

In an embodiment, the biostimulator 100 includes an attachment feature 210. The attachment feature 210 can be mounted on a proximal housing end 213 of the housing 202. More particularly, the attachment feature 210 can be mounted on an opposite end of the housing 202 from the fixation element 110 and the pacing element 108, which as described above, can be coupled to the distal housing end 209 of the housing 202. The attachment feature 210 can facilitate precise delivery or retrieval of the biostimulator 100. For example, the attachment feature 210 can be formed from a rigid material to allow a transport system to engage the attachment feature 210 and transmit torque and/or axial loads to the attachment feature 210 to plunge the fixation element 110 or the pacing element 108 into the target tissue.

The biostimulator 100 can include the pacing element 108 coupled to the housing 202. The pacing element 108 can be coaxially arranged with the fixation element 110 about the longitudinal axis 204. More particularly, the pacing element 108 can extend along, e.g., axially along or helically about, the longitudinal axis 204 at a location that is radially inward from the fixation element 110.

The pacing element 108 can, like the fixation element 110, affix or anchor in the target tissue. For example, the pacing element 108 may include a helical element extending distally to a distal tip having a distal pacing point. The distal pacing point of the pacing element 108 may be distal to a distal fixation tip of the fixation element 110. Both the fixation element 110 and the pacing element 108 can include a helical element to screw into a target tissue or a conical element to pierce into the target tissue. The fixation element 110 and/or the pacing element 108 can engage the tissue, and the housing 202 can be advanced and/or rotated to cause the distal pacing point of the pacing element to pierce the tissue and anchor the biostimulator 100. Accordingly, both the fixation element 110 and the pacing element 108 may be referred to as fixation elements, which are protected from the target tissue by a protective sleeve as described below.

Referring to FIG. 3, a perspective view of a biostimulator system in an undeployed state is shown in accordance with an embodiment. As described above, the biostimulator 100 can be delivered to or retrieved from a patient using delivery or retrieval systems. The delivery or retrieval systems, which may be catheter-based systems used to carry a leadless biostimulator intravenously to or from a patient anatomy. The delivery or retrieval systems may be referred to collectively as transport systems, or biostimulator transport systems.

A biostimulator system 300 can include a biostimulator transport system 302. The biostimulator 100 can be attached, connected to, or otherwise mounted on the biostimulator transport system 302. For example, the biostimulator transport system 302 can include a catheter shaft 304 extending to a distal shaft end 306, and the biostimulator 100 can be mounted on the catheter shaft 304. More particularly, the biostimulator transport system 302 can include a biostimulator coupling 308 mounted on the distal shaft end 306 to receive the biostimulator 100 having a fixation element (e.g., the fixation element 110 and/or the pacing element 108). The biostimulator 100 can thereby be advanced intravenously into or out of the heart 102.

The biostimulator transport system 302 can include a handle 310 to control movement and operations of the transport system from outside of a patient anatomy. One or more elongated members can extend distally from the handle 310. For example, the catheter shaft 304 can extend distally from the handle 310. The catheter shaft 304 can extend to the biostimulator coupling 308 at a distal end of the transport system.

In an embodiment, the biostimulator transport system 302 includes a sheath 312. The sheath 312 can have a distal sheath end 314. As described below, the biostimulator transport system 302 can further include a deployment tube 316, which can extend from the distal sheath end 314. More particularly, the deployment tube 316 can include a tubular extension or guide extending from the distal sheath end to a distal tube end 318. The sheath 312 and the deployment tube 316 can cover the biostimulator 100 and/or the biostimulator coupling 308 during delivery and implantation. For example, the sheath 312 and/or the deployment tube 316 can extend over, and be longitudinally movable relative to, the catheter shaft 304. Accordingly, the biostimulator 100 can be movable through the deployment tube 316 from within the sheath 312 or deployment tube 316 to a distal implantation site at the target tissue 106. As described below, location and orientation of the deployment tube 316 and, thus, the biostimulator 100 can be guided by an expandable structure having a target anatomy shaped, e.g., ventricle-shaped, envelope that conforms to the target anatomy.

The biostimulator transport system 302 may also include an introducer sheath 320 that can extend over, and be longitudinally movable relative to, the sheath 312. The introducer sheath 320 can cover a distal end of the sheath 312, the catheter shaft 304, the biostimulator coupling 308, and/or the biostimulator 100 as those components are passed through an access device into the patient anatomy.

Several components of the biostimulator transport system 302 are described above by way of example. It will be appreciated, however, that the biostimulator transport system 302 may be configured to include additional or alternate components. More particularly, the biostimulator transport system 302 may be configured to deliver and/or retrieve the biostimulator 100 to or from the target anatomy. Delivery and/or retrieval of the biostimulator 100 can include retaining the biostimulator during transport to the target anatomy and rotation of the biostimulator during implantation of the biostimulator at the target anatomy. For example, retention and/or rotation of the biostimulator 100 may be facilitated by the biostimulator coupling 308. Accordingly, the biostimulator transport system 302 can incorporate features to retain and rotate the biostimulator 100.

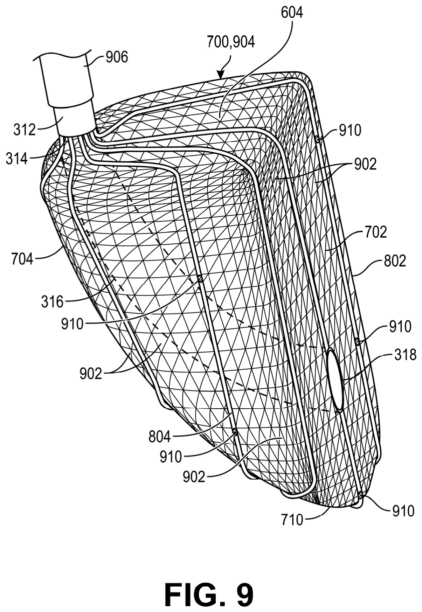

The biostimulator transport system 302 may incorporate a protective sleeve (FIG. 9) to cover at least a distal portion of the system. For example, the protective sleeve can cover all or a portion of the sheath 312 and/or the deployment tube 316. The protective sleeve, when covering the deployment tube 316, is in a protective and constraining state. For example, the distal positioning of the protective sleeve can constrain the expandable structure in an undeployed or unexpanded state. When the catheter is positioned near the target tissue, e.g., in the right ventricle, the protective sleeve can be pulled back to expose the expandable structure and to allow the expandable structure to expand to a deployed or expanded state. The biostimulator 100 may then be advanced to pierce the target tissue to anchor the biostimulator for pacing. Such function is introduced here and described in more detail below.

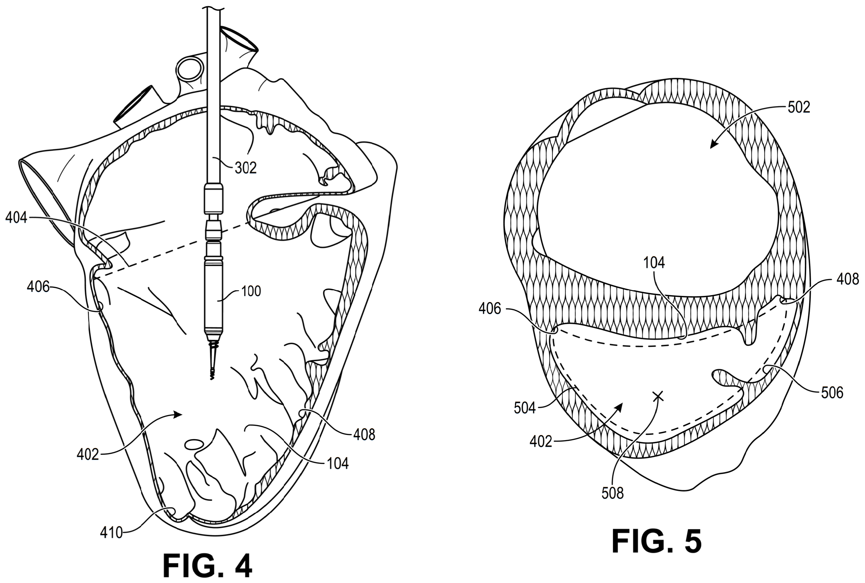

Referring to FIG. 4, a diagrammatic cross section of a patient heart illustrating an example delivery of a biostimulator 100 to a septal wall 104 is shown in accordance with an embodiment. A target anatomy, e.g., a right ventricle 402, can have anatomical landmarks around a periphery of the heart chamber. For example, a tricuspid valve region 404 can demarcate the chamber at an upper location, a posterior groove 406 can extend vertically along a rear side of the chamber, an anterior groove 408 can extend vertically along a front side of the chamber, and an apex region 410 can contain the chamber at a lower location. When viewing the chamber in a medial direction, the anatomical landmarks can frame the septal wall 104.

As described below, a biostimulator 100 can be advanced into the septal wall 104 by the biostimulator transport system 302. More particularly, the biostimulator 100 can be directed to a mid-septal area at a location and orientation that allows the pacing element 108 to engage the target tissue 106. Approaching the mid-septal area correctly can, however, be difficult to achieve using existing systems. For example, it has been discovered that the biostimulator 100 may inadvertently stray toward the chamber grooves or the apex region 410 using existing systems, missing the mid-septal landing zone.

Referring to FIG. 5, a diagrammatic cross section of a patient heart illustrating a ventricle shape is shown in accordance with an embodiment. An analysis of the ventricle 402, e.g., using 3D virtual models constructed from computerized tomography (CT) scans, reveals a profile defined by the anatomical landmarks of the ventricle 402. When the heart 102 is cross-sectioned along a transverse plane, e.g., a horizontal plane extending through the chamber of FIG. 4, the cross-section of the ventricle 402, e.g., a right ventricle, and an adjacent ventricle 502, e.g., a left ventricle, reveals characteristic shapes. Whereas the shape of the adjacent ventricle 502 is approximately round or oval-shaped, the ventricle 402 has tissue walls that define a profile having angular interfaces. More particularly, the ventricle 402 has a chamber profile 504 defined by the septal wall 104, the posterior groove 406, the anterior groove 408, and a free wall 506, and one or more of the angles formed between the septal wall 104 and the free wall 506 may be acute angle(s). The septal wall 104 can extend between the posterior groove 406 and the anterior groove 408 on a medial side of the ventricle. Similarly, the free wall 506 can extend between the posterior groove 406 and the anterior groove 408 on a lateral side of the ventricle. The chamber profile 504 can trace along the walls and, as shown, can extend around a central chamber axis 508.

In an embodiment, the chamber profile 504 has a crescent shape. For example, relative to the central chamber axis 508, the septal wall 104 can be concave away and the free wall 506 can be convex away. The walls may further meet at edges adjacent to the posterior and anterior grooves. The crescent-shape of the chamber profile 504 can result from pressures in the adjacent ventricle 502 being higher on average than the pressures in the ventricle 402. A target location for the biostimulator 100 can be approximately mid-septum, e.g., midway between the apex region 410 and the basal grooves, and midway between the posterior groove 406 and the anterior groove 408. The convex nature of the septal wall 104 can, however, complicate delivery to the target location because the domed surface can direct a catheter or biostimulator 100 laterally to the anterior or posterior groove during advancement. Precise placement of the biostimulator 100 at the mid-septum can be critical, however, because penetrating the myocardium in the posterior and anterior grooves can miss the target tissue 106 on the septum and/or perforate the free wall 506.

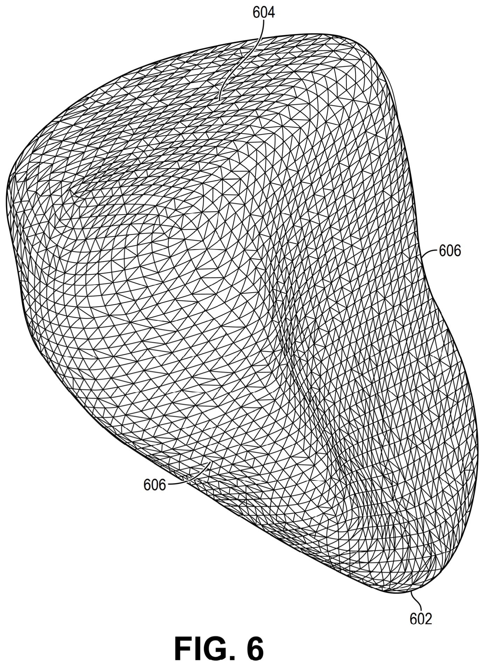

Referring to FIG. 6, a pictorial view of a virtual model of a ventricle shape is shown in accordance with an embodiment. A 3D virtual model of the ventricle 402 can be constructed as a solid model of space inside the heart chamber, e.g., below the papillary muscles and tricuspid valve chordae, and above the apex region 410. The virtual model may, for example, represent an amalgamation of computerized tomography (CT) scans from more than one hundred patients representative of a varied patient population. The virtual model can define a chamber envelope having a bottom apex 602, a top surface 604, and several sidewalls 606 extending upward between the bottom apex 602 and the top surface 604. Notably, the sidewalls 606 can include a front boundary having a concave surface conforming to the representative septal wall and a rear boundary having a convex surface conforming to the representative free wall.

In an embodiment, the ventricle-shaped envelope 904 has, in the expanded state, a crescent-shaped cross-sectional profile 808 representing a right ventricle 404 of a heart 102. This crescent-shaped cross-sectional profile 808 represents, in an embodiment, a template or reference right ventricle 404, such as obtained from an amalgamation of CT scans from several patients. The crescent-shaped cross-sectional profile 808 may, thus, represent a population-based or canonical right ventricle 404.

The ventricular shape may, as described below, be used to urge the biostimulator 100 or the biostimulator transport system 302 to the target location on the septal wall 104, rather than toward the non-advantageous positions along the anterior and posterior grooves. More particularly, the virtual model can be mimicked by an expandable structure that supports the deployment tube 316 in the ventricle 402 to direct the biostimulator 100 toward the mid-septum location on the septal wall 104.

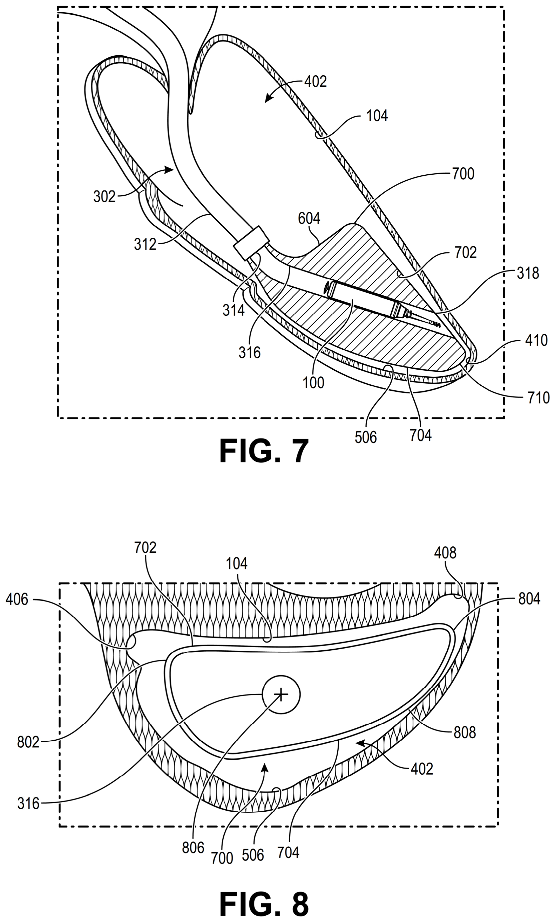

Referring to FIG. 7, a diagrammatic cross section of a patient heart illustrating an example delivery of a biostimulator through an expandable structure of a biostimulator transport system is shown in accordance with an embodiment. The biostimulator transport system 302 can include an expandable structure 700 coupled to the distal sheath end 314 of the sheath 312. In an embodiment, the expandable structure 700 defines an envelope surrounding the deployment tube 316 in the expanded state. More particularly, the expandable structure 700 can have a proximal location coupled to the distal sheath end 314 and a distal location coupled to the distal tube end 318, and the deployment tube 316 can extend through an internal space defined by the envelope of the expandable structure 700 between the distal sheath end 314 and the distal tube end 318. The expandable structure 700 may therefore surround and support the deployment tube 316 within the ventricle 402. More particularly, the expandable structure 700 can expand outward into contact with the ventricular walls to stabilize the deployment tube 316 within the ventricle 402. The biostimulator 100 may be advanced through the deployment tube 316 toward the mid-septum implant location on the septal wall 104.

In an embodiment, the expandable structure 700 has a ventricle-shaped envelope surrounding the deployment tube 316. The ventricle-shaped envelope can be constructed such that it approximates or duplicates the shape or volume of the 3D virtual model described above. For example, the ventricle-shaped envelope can be wedge-shaped, extending from a wider top surface 604 to a narrower bottom edge 710. The bottom edge 710 can be a point of the wedge, extending distally away from the distal sheath end 314. More particularly, lateral surfaces of the envelope can taper inward from the top surface 604 to the bottom edge 710. The ventricle-shaped envelope can have a front boundary 702 that approximates a shape of the septal wall 104, and a rear boundary 704 that approximates a shape of the free wall 506. The front boundary 702 can be coupled to the distal tube end 318, and the rear boundary 704 can be coupled to the distal tube end 318. Accordingly, when the expandable structure 700 is deployed, e.g., expanded into the expanded state, in the ventricle 402, the front boundary 702 can appose and conform to the septal wall 104 and the rear boundary 704 can appose and conform to the free wall 506. The attachments between the boundaries and the ends of the sheath 312 and deployment tube 316 can create a framework or structure that supports the deployment tube 316 within the heart chamber and holds the distal tube end 318 at the mid-septum location.

Referring to FIG. 8, a diagrammatic cross section of a patient heart illustrating an example delivery of an expandable structure of a biostimulator transport system 302 in a ventricle 402 is shown in accordance with an embodiment. Similar to the conformance of the expandable structure 700 to the sidewalls 606 of the ventricle 402, the expandable structure 700 may also have a cross-sectional profile that approximate the chamber profile 504 of the ventricle 402.

The front boundary 702 of the expandable structure 700 and the rear boundary 704 of the expandable structure 700 can be joined along peripheral or lateral edges that fit in the grooves of the target heart chamber. For example, the front boundary 702 can meet the rear boundary 704 at a posterior lateral edge 802 that fits in the posterior groove 406 when the expandable structure 700 is deployed in the ventricle 402. Similarly, the front boundary 702 can meet the rear boundary 704 at an anterior lateral edge 804 that fits in the anterior groove 408 when the expandable structure 700 is deployed in the ventricle 402. The lateral edges can preferentially orient into the ventricular grooves during expansion to cause the front boundary 702 to appose the septal wall 104.

Referring again to FIG. 7, the front boundary 702 and the rear boundary 704 can meet at a bottom edge 710. More particularly, the expandable structure 700 can include the bottom edge 710 at which the vertically extending boundaries converge. When the expandable structure 700 is expanded within the ventricle 402, the bottom edge 710 can deploy into the apex region 410 of the ventricle 402. Accordingly, the lateral edges can engage the ventricular grooves and the bottom edge 710 can engage the apex region 410 to stabilize the expandable structure 700, and the deployment tube 316, within the ventricle 402.

Referring again to FIG. 8, the front boundary 702 can have a concave or a substantially flat surface that presses against and conforms to the mid-septum region of the septal wall 104. Similarly, the rear boundary 704 can have a curved surface that conforms to the free wall 506. In an embodiment, the concave and convex curvatures of the expandable structure 700 define a crescent-shape. More particularly, a plane extending orthogonal to a central axis 806 of the deployment tube 316 can reveal the cross-sectional profile shown in FIG. 8, which may be a crescent-shaped cross-sectional profile 808 of the ventricle-shaped envelope of the expandable structure 700. The crescent-shaped cross-sectional profile 808 can include the curvilinear portion corresponding to the rear boundary 704, which may be convex relative to the central axis 806 of the deployment tube 316. Furthermore, the crescent-shaped cross-sectional profile 808 can include the curvilinear portion corresponding to the front boundary 702, which may be concave relative to the central axis 806 of the deployment tube 316.

In an embodiment, the curvatures of the front boundary 702 and the rear boundary 704 may be defined relative to each other. For example, the front boundary 702 may be flatter than the rear boundary 704 and have a corresponding difference in radius. More particularly, a radius of the curvature of the front boundary 702 can be larger than a radius of the curvature of the rear boundary 704 (in the sense that a radius of a line is infinite). The curves can therefore combine to form the crescent shape that conforms to the average ventricular shape corresponding to the virtual model.

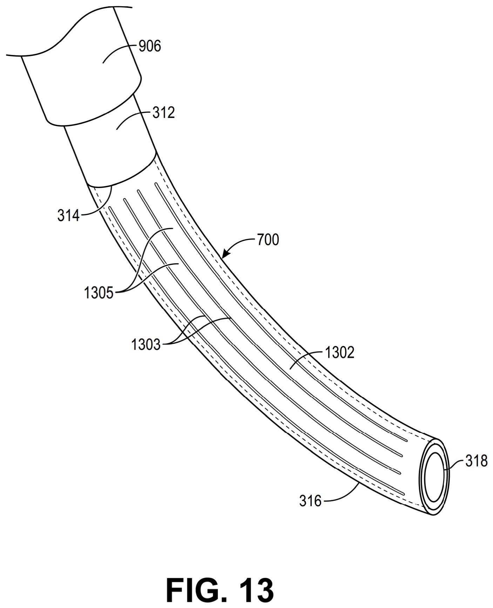

Several embodiments of the expandable structure 700 are described below. In each embodiment, the expandable structure 700 can deploy from an unexpanded state (e.g., FIG. 13) in which the front boundary 702 and the rear boundary 704 are collapsed around the deployment tube 316, to an expanded state (e.g., FIGS. 7-12) in which the lateral edges are laterally offset from the distal tube end 318. For example, the anterior lateral edge 804 and the posterior lateral edge 802 can extend vertically and be separated from each other across the front boundary 702. In the expanded state, the lateral edges can be laterally offset from the distal tube end 318. For example, the distal tube end 318 can be midway between the lateral edges. Accordingly, when the expandable structure 700 is deployed in the ventricle 402 and the lateral edges engage the ventricular grooves, the distal tube end 318 can be located at the mid-septum location.

Although the expandable structure 700 can partially fill the ventricle 402 in the expanded state, the envelope of the expandable structure 700 may not occupy the entire ventricle 402. For example, the bottom edge 710 can engage the apex region 410 and the crescent-shaped cross-sectional profile 808 may substantially conform to the vertical walls of the heart chamber, however, as shown in FIG. 7 a top surface 604 of the expandable structure 700 may stop short of the papillary muscles at the free wall 506 to avoid interfering with the tricuspid valve. More particularly, a top portion of the expandable structure 700 can be at or just below the papillary muscles in the ventricle 402 when the expandable structure 700 is deployed in the ventricle 402.

Referring to FIG. 9, a perspective view of a biostimulator transport system in an expanded state is shown in accordance with an embodiment. The expandable structure 700 can include a self-expandable structure 700. For example, the expandable structure 700 may include shape memory alloy wires formed into a structure, e.g., a framework, that approximates the ventricular shape, making a ventricle-shaped envelope 904.

In an embodiment, the expandable structure 700 includes a braid 902 extending from the distal sheath end 314 to the distal tube end 318. For example, the distal sheath end 314 can be attached to the expandable structure 700 at a location along a top surface 604 of the expandable structure 700 near the rear boundary 704, opposite the front boundary 702. The deployment tube 316 may also extend from the distal sheath end 314, and can pass through an interior of the braided framework to connect to the front boundary 702 of the framework. For example, the deployment tube 316 can include a solid tubular element to direct the biostimulator 100 distally from the distal sheath end 314 to the distal tube end 318. The passage provided by the deployment tube 316, which is indicated by hidden lines, can be fixed to the front boundary 702 to provide a stabilized port through which the biostimulator 100 can be advanced into the target tissue 106.

The braided structure can include an expandable, tubular braid. For example, in the unexpanded state, the braided structure can be a tubular braid having a proximal tubular end coupled to the distal sheath end 314 and a distal tubular end at the distal tube end 318. The expanded state of the tubular braid can have a ventricle-shaped envelope 904. The expandable structure 700 can transition between the expanded state and the unexpanded state by retracting the structure into a catheter lumen, e.g., inside of the sheath 312 or the protective sleeve 906. Conversely, the expandable structure 700 can transition between the unexpanded state and the expanded state by advancing the expandable sheath 312 from the catheter lumen to cause the structure to recover to the expanded envelope. The structure can be attached to the deployment tube 316, which may be slidable within the catheter lumen, e.g., within the sheath 312 or the protective sleeve 906, to move the distal tube end 318 relative to the distal sheath end 314 and retract or advance the expandable structure 700 from the catheter lumen.

Transitioning between the unexpanded state and the expanded state may result from a shape memory characteristic of the braid 902. For example, the structure can include elastic, formed wires, e.g., nickel-titanium alloy wires, or other shape memory wires that are braided and bound together. The structure can be heat set in such a manner that the braid 902 in the expanded state has the ventricle-shaped envelope 904. In an embodiment, the braid 902 can be pulled into a protective sleeve 906 to collapse the framework into the unexpanded state. Accordingly, the expandable structure 700 can be deployable from the unexpanded state, in which the front boundary 702 and the rear boundary 704 are collapsed around the deployment tube 316, to the expanded state, in which the lateral edges, e.g., the posterior lateral edge 802 and the anterior lateral edge 804 expand laterally outward. In the deployed state, the braid 902 can take on the expanded braid 902 shape having the ventricle-shaped envelope 904. When deployed in the ventricle 402, the lateral edges can engage anatomical landmarks, such as the ventricular grooves, to stabilize and position the distal tube end 318 at the mid-septum.

In an embodiment, the expandable structure 700 include one or more radiopaque markers 910. For example, the radiopaque marker(s) can be located along the bottom edge 710 and/or the lateral edges 802, 804 of the ventricle-shaped envelope 904. Optionally, the radiopaque markers 910 can be located in other regions, such as along the free wall 506. The markers can provide visual feedback regarding a position of the structural elements when the expandable structure 700 is deployed in the ventricle 402. For example, a user may view the expanded structure within the ventricle 402 under fluoroscopy to confirm that the bottom edge 710 is engaging the apex region 410 and the lateral edges are engaging the ventricular grooves, indicating that the expandable structure 700 is properly seated and the distal tube end 318 is position near the mid-septum.

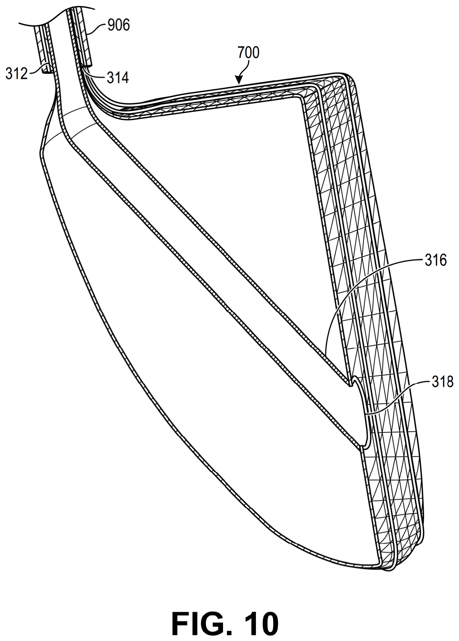

Referring to FIG. 10, a perspective cross-sectional view of a biostimulator transport system in an expanded state is shown in accordance with an embodiment. The deployment tube 316 can originate at the distal sheath end 314 and extend through the interior of the expandable structure 700 at a bias toward the distal tube end 318. A proximal portion of the deployment tube 316 can attach to the distal sheath end 314 of the sheath 312, or may be movable through the sheath 312. Similarly, the deployment tube 316 may be slidable through the protective sleeve 906. In either case, the distal tube end 318 can be movable relative to the protective sleeve 906 and, optionally, movable relative to the distal sheath end 314.

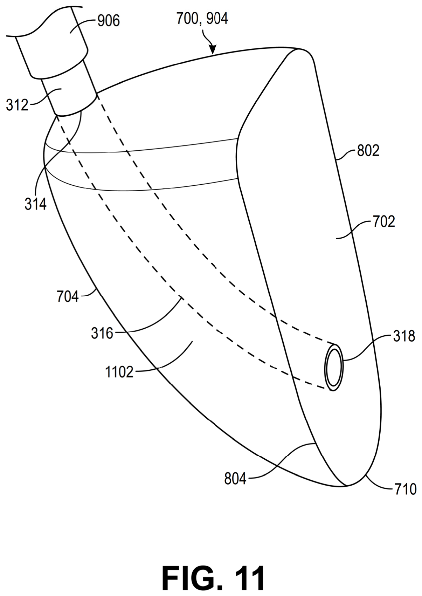

Referring to FIG. 11, a perspective view of a biostimulator transport system in an expanded state is shown in accordance with an embodiment. The expandable structure 700 may be inflatable. More particularly, the expandable structure 700 can include an inflatable element 1102, such as a balloon, having the ventricle-shaped envelope 904 in the expanded state. The inflatable element 1102 can extend from the distal sheath end 314 to the distal tube end 318, and the deployment tube 316 can travel through an interior of the inflatable element 1102, e.g., at the bias described above.

The inflatable element 1102 can include the balloon (or balloons) having a compliant, semi-compliant, or non-compliant structure. More particularly, the balloon(s) can be shaped and fabricated to cause the inflatable element 1102 to assume the shape of the ventricle 402 when inflated. Accordingly, the inflatable element 1102 can have the front boundary 702, rear boundary 704, bottom edge 710, and lateral edges, as described above. By contrast, when the inflatable element 1102 is deflated, a membrane, e.g., a balloon wall, of the inflatable element 1102 can collapse around the deployment tube 316 and be pulled into the catheter lumen of the sheath 312 or the protective sleeve 906.

It will be appreciated that the inflatable element 1102 may displace volume within the heart chamber, which could be disadvantageous to heart function. In an embodiment, the inflatable element 1102 is shaped to reduce the displaced volume. For example, the inflatable element 1102 may have one or more lobes or wings radiating laterally from the deployment tube 316. The lobes can be separated by gaps, as shown in FIG. 11, which provide space for blood to flow through when the inflatable element 1102 is inflated. Accordingly, the lobes can support the deployment tube 316 while supporting normal heart function.

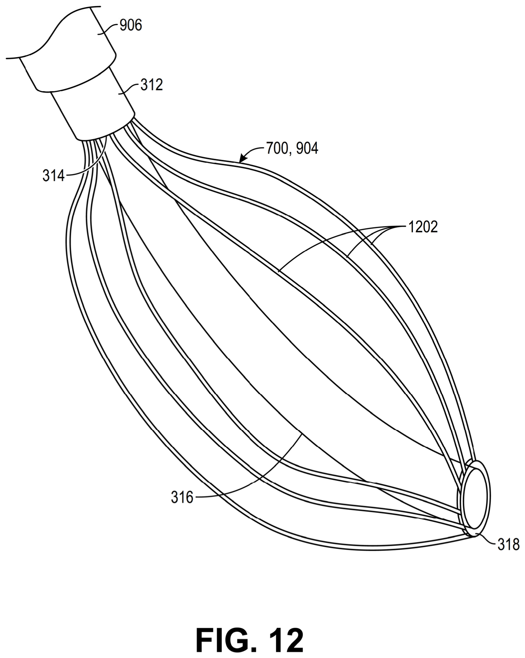

Referring to FIG. 12, a perspective view of a biostimulator transport system in an expanded state is shown in accordance with an embodiment. The expandable structure 700 having the ventricle-shaped envelope 904 can include several elastic wires 1202. The elastic wires 1202 may have shape memory, or may be forced into the expanded state by compression. In an embodiment, the elastic wires 1202 extend from the distal sheath end 314 to the distal tube end 318 and, at least in the expanded state, bow outward to form an overall envelope having the ventricle shape.

The structure formed by the elastic, formed wires can be constructed and bound together, e.g., formed, heat set, etc., similar to the braid 902 described above. More particularly, the wires can be heat set in the ventricle shape. The wires may be more sparse than the braid 902, and therefore may provide less wall coverage of the heart chamber. However, the wires may have a stiffness that adequately supports the deployment tube 316 and positions the distal tube end 318 at the mid-septum without interfering with chordae or other ventricular structures.

Referring to FIG. 13, a perspective view of a biostimulator transport system in an unexpanded state is shown in accordance with an embodiment. The expandable structure 700 may include a slotted tube 1302 extending from the distal sheath end 314 to the distal tube end 318. The slotted tube 1302 can include a hypotube formed from an elastic material, such as a nickel-titanium alloy. The hypotube can have several slots 1303, e.g., directed axially along the tube, spiral cut, etc., that forms adjacent ribs 1305. When the ribs 1305 are compressed, e.g., by moving the distal tube end 318 relative to the distal sheath end 314 and thereby compressing the deployment tube 316 therebetween, the ribs can expand outward to the shape illustrated in FIG. 12. More particularly, the ribs 1305 can act like elastic wires 1202 that take on the ventricle-shaped envelope 904 described above. When the deployment tube 316 is retracted to compress and expand the slotted tube 1302, the deployment tube 316 can extend through the interior of the ventricle-shaped enveloped and may be stabilized by the expanded structure to direct the distal tube end 318 to the mid-septum.

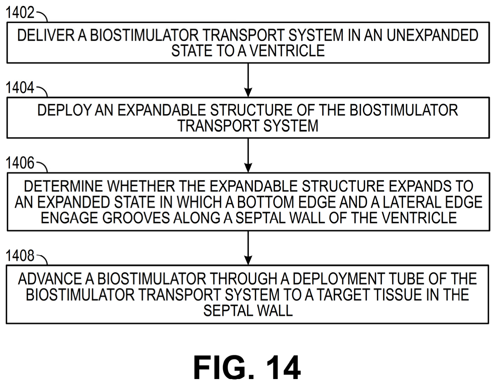

Referring to FIG. 14, a flowchart of a method of implanting a biostimulator in a target tissue is shown in accordance with an embodiment. The biostimulator transport system 302 can be used to deliver the biostimulator 100 to the mid-septum region of the septal wall 104. At operation 1402, the biostimulator transport system 302 can be delivered into the ventricle 402 in the unexpanded state. More particularly, the expandable structure 700 can be delivered in the unexpanded state to the ventricle 402.

During delivery, the expandable structure 700 can be retracted into the catheter shaft 304 to compress the structure (or may be in a state of tension in the case of the slotted tube 1302). In any case, the expandable structure 700 can fit within the catheter lumen and can be collapsed around the biostimulator 100.

At operation 1404, the expandable structure 700 is deployed. When the expandable structure 700 is placed in the ventricle 402, the structure can be expanded. Some embodiments include advancement of the expandable structure 700 from the catheter lumen, e.g., from the protective sleeve 906 or from the sheath 312. For example, deploying the expandable structure 700 can include sliding the deployment tube 316 through the sheath 312 to move the distal tube end 318 relative to the distal sheath end 314, allowing the expandable structure 700 to become unconstrained and expand. Alternatively, the expandable structure 700 can be compressed to cause the structure to expand, or may be inflated. In any case, the expandable structure 700 can be expanded to reside in the ventricle 402 with the outer surface of the expandable structure 700 being in contact with the ventricular wall. When the expandable structure 700 is properly seated, the deployment tube 316 can be directed to a target implant site. More particularly, the deployment tube 316 can be supported and oriented within the ventricle 402 with the central axis 806 of the tube being at an appropriate angle with the septal wall 104 and at an appropriate location near the mid-septum to allow the biostimulator 100 to be advanced to a proper depth and ensure that appropriate therapy is delivered. Furthermore, such placement can reduce a likelihood of cardiac perforation or other tissue damage.

At operation 1406, it is determined whether the expandable structure 700 expands to the expanded state in which the bottom edge 710 and the lateral edges engage the appropriate anatomical landmarks. For example, a user or vision system may determine whether the bottom edge 710 and the lateral edges engage grooves along the septal wall 104 of the ventricle 402. Such confirmation can be provided by viewing the expandable structure components, e.g., the wires or radiopaque markers 910, under fluoroscopy. When the expandable structure 700 has expanded to the expected shape and location, the user can be confident that the shape-directed structure is properly deployed and the distal tube end 318 is at the mid-septum (not too high nor too low on the septal wall 104), not in or near the anterior or posterior septal grooves, and that the deployment tube 316 is at a correct angle (not too acute or too obtuse). If the structure is not fully or properly deployed, the structure may be partially collapsed and re-deployed until visualization confirms proper placement.

At operation 1408, when it has been confirmed that the expandable structure 700 is properly located and/or oriented, the biostimulator 100 can be advanced through the deployment tube 316 to the target tissue 106 in the septal wall 104. The pacing element 108 and/or fixation element 110 can be moved into contact with the septal wall 104, and driven into the target tissue 106. For example, the biostimulator 100 may be rotated to screw the fixation element 110 into the septal wall 104 and advance the pacing element 108 to the target pacing site, e.g., the left bundle branch. The biostimulator 100 may then be used to provide pacing therapy.

In the foregoing specification, the invention has been described with reference to specific exemplary embodiments thereof. It will be evident that various modifications may be made thereto without departing from the broader spirit and scope of the invention as set forth in the following claims. The specification and drawings are, accordingly, to be regarded in an illustrative sense rather than a restrictive sense.

## Claims

1. A biostimulator transport system (302), comprising:
a sheath (312) having a distal sheath end (314);
a deployment tube (316) extending from the distal sheath end (314) to a distal tube end (318); and
an expandable structure (700) having a ventricle-shaped envelope (904) in an expanded state, wherein the ventricle-shaped envelope (904) surrounds the deployment tube (316), wherein the ventricle-shaped envelope (904) includes a front boundary (702) coupled to the distal tube end (318) and a rear boundary (704) coupled to the distal sheath end (314), and wherein the front boundary (702) and the rear boundary (704) are joined along one or more of a bottom edge (710) or lateral edges (802, 804).

2. The biostimulator transport system of claim 1, wherein the expandable structure (700) is deployable from an unexpanded state in which the front boundary (702) and the rear boundary (704) are collapsed around the deployment tube (316) to the expanded state in which the lateral edges (802, 814) are laterally offset from the distal tube end (318).

3. The biostimulator transport system of claim 1 or 2, wherein the deployment tube (316) is slidable within the sheath (312) to move the distal tube end (318) relative to the distal sheath end (314).

4. The biostimulator transport system of any one of claims 1 to 3, wherein the ventricle-shaped envelope (904) has, in the expanded state, a crescent-shaped cross-sectional profile (808) taken along a plane extending orthogonal to a central axis (806) of the deployment tube (316).

5. The biostimulator transport system of claim 4, wherein the crescent-shaped cross-sectional profile (808) includes a rear curvilinear portion corresponding to the rear boundary (704).

6. The biostimulator transport system of claim 4 or 5, wherein the crescent-shaped cross-sectional profile (808) includes a front curvilinear portion corresponding to the front boundary (702).

7. The biostimulator transport system of claim 6 when dependent on claim 5, wherein a front radius of curvature of the front curvilinear portion is greater than a rear radius of curvature of the rear curvilinear portion.

8. The biostimulator transport system of any one of claims 1 to 7, wherein the rear boundary (704) is convex relative to a central axis (806) of the deployment tube (316).

9. The biostimulator transport system of any one of claims 1 to 8, wherein the front boundary (702) is concave relative to a central axis (806) of the deployment tube (316).

10. The biostimulator transport system of any one of claims 1 to 9, wherein the expandable structure (700) includes a plurality of elastic wires (1202) extending from the distal sheath end (314) to the distal tube end (318).

11. The biostimulator transport system of any one of claims 1 to 10, wherein the expandable structure (700) includes a braid (902) extending from the distal sheath end (314) to the distal tube end (318).

12. The biostimulator transport system of any one of claims 1 to 10, wherein the expandable structure (700) includes a slotted tube (1302) extending from the distal sheath end (314) to the distal tube end (318).

13. The biostimulator transport system of any one of claims 1 to 12, wherein the expandable structure (700) includes an inflatable element (1102) extending from the distal sheath end (314) to the distal tube end (318).

14. The biostimulator transport system of any one of claims 1 to 13, further comprising one or more radiopaque markers (910) along the bottom edge (710) and the lateral edges (802, 804).

15. A biostimulator system (300), comprising:
the biostimulator transport system (302) of any one of claims 1 to 14; and
a biostimulator (100) movable through the deployment tube (316), wherein the biostimulator (100) includes a body (103) having an electronics compartment (206) containing circuitry.
